# EUROPÄISCHE PATENTSCHRIFT

(11) **EP 0 670 708 B1**
(45) Veröffentlichungstag und Bekanntmachung des Hinweises auf die Patenterteilung: **04.09.1996**
(21) Anmeldenummer: 94900797.5
(22) Anmeldetag: 10.11.1993
(51) Int. Cl.: A61G 9/02, F15D 1/08, B67C 3/26, A47L 15/14, A61L 2/18

(54) **VORRICHTUNG ZUM SPÜLEN UND/ODER DESINFIZIEREN VON PFLEGEGESCHIRREN**
DEVICE FOR WASHING AND/OR DISINFECTING MEDICAL WARE
DISPOSITIF POUR LE LAVAGE ET/OU LA DESINFECTION D'USTENSILES DE SOIN

(30) Priorität: 23.11.1992 DE 9215876 U
(43) Veröffentlichungstag der Anmeldung: 13.09.1995
(73) Patentinhaber: Discher, Josef, D-42781 Haan (DE)
(72) Erfinder: Discher, Josef, D-42781 Haan (DE)
(74) Vertreter: Rieder, Hans-Joachim, Dr.
(86) Internationale Anmeldenummer: EP9303140
(87) Internationale Veröffentlichungsnummer: WO9412139

(56) Entgegenhaltungen:
- CH-A- 625 699
- DE-A- 2 900 957
- DE-A- 4 107 578
- FR-A- 2 294 687
- US-A- 4 670 061

## Beschreibung

Die Erfindung betrifft eine Vorrichtung zum Spülen und/oder Desinfizieren von Pflegegeschirren, insbesondere Bett-Pflegegeschirren, mit einer das Pflegegeschirr aufnehmenden Kammer und einem dieser vorgeordneten Wasservorratsbehälter, dem ein mit dem Wasserleitungsnetz verbindbarer Zulauf zugeordnet ist, wobei die Strahlaustrittsmündung in keiner Flächenverbindung steht zur wasserbenetzten Innenwand des Wasservorratsbehälters.

Eine Vorrichtung der in Rede stehenden Art ist bekannt aus der FR-A-2 294 687, bei welcher das Wasserzuflußrohr eine Durchtrittsöffnung des Wasservorratsbehälters durchsetzt und mit der abwärts gewinkelten Strahlaustrittsmündung oberhalb des Wasserspiegels des Wasservorratsbehälters endet: Diese Bauform ist nicht ausreichend gebrauchssicher. Es besteht nämlich die Möglichkeit, daß Bakterien in die Verbrauchs- und Versorgungsleitungen des Wasserversorgungsnetzes hineingeraten können. So kann sich an der die Durchtrittsöffnung aufweisenden Innenwand eine Bakterienkolonie aufbauen. Diese kann sich ringsherum um die Durchtrittsöffnung fortsetzen. Da bei dieser Vorrichtung sowohl mit Kalt- als auch Warmwasser gearbeitet wird, ist auch mit einer Kondensation in dem Wasservorratsbehälter zu rechnen verbunden mit einem Abregnen von eventuell Bakterien aufweisenden Wassertropfen, die dann auch auf das Wasserzuflußrohr gelangen können. Es besteht demnach die Möglichkeit, daß sich auch dort Bakterien ansiedeln und danach in die Mündungsöffnung des Zuflußrohres hineinwachsen und damit in die Verbrauchs- und Versorgungsleitungen gelangen.

Dem Gegenstand der Erfindung liegt daher die Aufgabe zugrunde, eine Vorrichtung der in Rede stehenden Art in herstellungstechnisch einfacher Weise so auszugestalten, daß der Eintritt von Bakterien aus dem Wasservorratsbehälter in das Wasserleitungsnetz mit Sicherheit verhindert ist.

Gelöst wird diese Aufgabe durch einen freien Strahl als Zufluß zum Wasservorratsbehälter, derart, daß die Strahlaustrittsmündung über der Decke des Wasservorratsbehälters angeordnet ist und daß die Decke (40) eine Einflußöffnung aufweist.

Zufolge derartiger Ausgestaltung ist eine Vorrichtung der in Rede stehenden Art geschaffen, welche den Eintritt von Bakterien aus dem Wasservorratsbehälter in das Wasserversorgungsnetz mit Sicherheit ausscheidet. Die Strahlaustrittsmündung ragt nun nicht mehr in den Wasservorratsbehälter hinein, sondern ist so positioniert, daß der freie Strahl als Zufluß zum Wasservorratsbehälter auftritt. Hierdurch wird das Problem eliminiert, daß abregnendes, Bakterien enthaltendes Kondenswasser in Berührung gelangt mit der Strahlaustrittsmündung wie beim Stand der Technik. Es läßt sich ferner bei gleichgroßer Bauform ein größeres Wasservolumen verwirklichen, da keine Durchtrittsöffnung in der seitlichen Behälterwandung vorhanden ist, unterhalb welcher Durchtrittsöffnung der Wasserspiegel liegen muß. Sodann arbeitet die Vorrichtung auch dann noch vorschriftsmäßig und mit großer Sicherheit, wenn nur ein geringer Wasserdruck vorliegen sollte. Der Wasserstrahl gelangt stets bestimmungsgemäß bei Öffnung des entsprechenden Zulaufventils schwerkraftunterstützt in den Wasservorratsbehälter.

Eine vorteilhafte Weiterbildung besteht darin, daß die Einflußöffnung der Decke sich als nach innen gerichteter Trichter fortsetzt. Dieser vermeidet, daß etwaige Wasserspritzer aus dem Wasservorratsbehälter austreten können.

Die Parallelausrichtung des Strahles ist dadurch begünstigt, daß im Zentrum des Strahles ein Strahlleitkörper, vorzugsweise in Form eines Borstenbüschels, angeordnet ist. Bei einem normal großen Leitungsanschluß kann dieses Borstenbüschel einen Durchmesser von etwa 3 mm besitzen. Als Borsten eignen sich insbesondere Nylonfäden, die an der Borstenwurzel untereinander verbunden sind.

Es ist dabei vorgesehen, daß der Strahlleitkörper an dem verschieblichen, vom einfließenden Wasser umspülten Ventilteller sitzt. Einhergehend mit einer Verlagerung desselben durch zuströmendes Wasser verändert der Strahlleitkörper seine Position. Wird der Leitungsdruck größer, bildet der Strahlleitkörper eine Verlängerung zwischen Strahlaustrittsmündung und dem Wasservorratsbehälter.

Damit die Strahlaustrittsmündung in keiner Flächenverbindung steht zur wasserbenetzten Innenwand des Wasservorratsbehälters, ist die Strahlaustrittsmündung an der Unterseite eines Galgens angeordnet, dessen Innenraum als Mischkammer des getrennt zugeführten warmen und kalten Wassers dient. Hierdurch erfüllt der Galgen sogar eine Doppelfunktion: einerseits weist er die Strahlaustrittsmündung an seiner Unterseite auf und andererseits dient sein Innenraum als Mischkammer des getrennt zugeführten warmen und kalten Wassers.

Eine homogene Durchmischung des getrennt zugeführten warmen und kalten Wassers erhält man insbesondere dadurch, daß die Zulaufstutzen am Galgen einander gegengerichtet und aus einer Gegenüberlage versetzt angeordnet sind.

Um nach Zudrehen der Absperrventile für das Warm- und Kaltwasser eine weitgehende Entleerung der vom Galgen gebildeten Mischkammer zu erreichen, verläuft der Galgen in Richtung der Strahlaustrittsmündung schwach geneigt.

Der im Wasservorratsbehälter vorgesehene Niveauhöhenregler sorgt dafür, daß bei Erreichen einer bestimmten Füllstandshöhe der Wasserzulauf abgeschaltet wird.

Gemäß der Erfindung ist der Niveauhöhenregler in einem abgeteilten Eckbereich des Wasservorratsbehälters angeordnet, der in Bodennähe einen Durchlaßquerschnitt zum abgeteilten Eckbereich beläßt. Auf diese Weise ist der Füllstand im abgeteilten Eckbereich sowie dem Restbereich des Wasservorratsbehälters stets gleich aufgrund des Prinzips kommunizierender Röhren. Auch befindet sich der Niveauhöhenregler in dem abgeteilten Eckbereich in einer geschützten Lage.

Zwecks Durchführung einer dezentralen chemischen Desinfektion ist am Wasservorratsbehälter vorzugsweise auf Höhe des mittleren gesteuerten Niveaus eine Zumischkammer angeflanscht, welch einen Zufluß für ein Desinfektionsmittel besitzt und einen Abfluß, der zu einer in der Kammer vorgesehenen Desinfektions-Sprüheinrichtung führt. Der Zufluß für das Desinfektionsmittel befindet sich vorzugsweise an der Oberseite der Zumischkammer. Dort kann auch ein Abluftstutzen vorgesehen sein, der in Leitungsverbindung steht zum Wasservorratsbehälter. Die mit dem als Luftstutzen in Verbindung stehende Leitung mündet oberhalb des obersten gesteuerten Niveaus. Der dritte Anschluß stellt den Abfluß dar, der unterhalb der Zumischkammer vorgesehen ist. Sowohl der Zufluß von Desinfektionsmittel als auch der Abfluß wird durch Pumpen gesteuert. Grundsätzlich ist erreicht, daß bei Füllung des Wasservorratsbehälters stets auch die Zumischkammer mit Wasser gefüllt ist.

Ein weiterer Vorteil der erfindungsgemäßen Vorrichtung ist darin zu sehen, daß die in waagerechte Richtung öffenbare Klappe der Kammer achsgelagerte Seitenholme besitzt, die über die Achslagerstelle ragende Anschlagabschnitte ausbilden, welche gegen die untere Kante einer Randeinfassung der frontseitigen Öffnung der Kammer schwenken. Die Anschlagabschnitte in Verbindung mit den unteren Schmalkanten erlauben schadfreie Belastungen der geöffneten Klappe der Kammer, welche Belastung beispielsweise eine sich auf die Klappe stellende Person sein kann. Bei in Betrieb befindlichen Vorrichtungen hat es sich nämlich gezeigt, daß zur Vergrößerung der Reichhöhe Personen sich auf die Klappe gestellt haben, um besser zu oberhalb der Vorrichtung angeordneten, aufgehängten Schränken zu gelangen. Unter dem Gewicht der Personen haben sich dabei Verformungen an der Klappe ergeben.

Eine labyrinthartige Dichtung bei gechlossener Klappe wird dadurch erzielt, daß die Randeinfassung einen Einschwenkquerschnitt für eine Randleiste der Klappe ausbildet und, bis zu den Schmalkanten reichend, an den freien unteren Enden verstärkt ist. Durch die Verstärkung vergrößert sich die Anlagefläche für die Anschlagabschnitte der Seitenräume.

Insgesamt ergibt sich eine besonders stabile Klappenausbildung dadurch, daß die Seitenräume Bestandteil eines Klappenrahmens sind. Letzterer ist auch Träger der Achse, so daß im Grunde genommen die Belastungen in den Klappenrahmen geleitet werden.

Nachstehend wird ein Ausführungsbeispiel der Erfindung anhand der Zeichnungen erläutert.

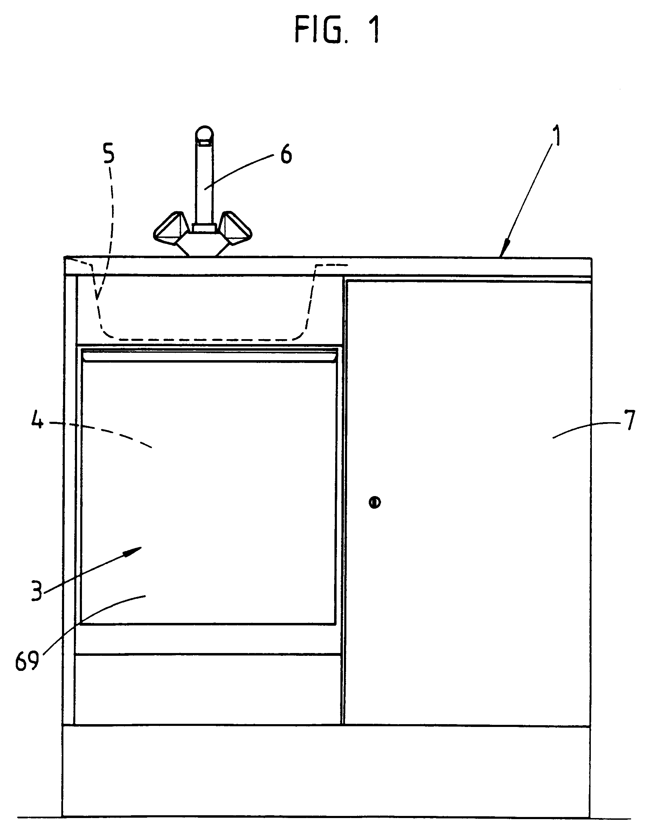

Es zeigt
- Figur 1: eine Vorderansicht einer in Form einer Spüle gestalteten Vorrichtung zum Spülen und Desinfizieren von Pflegegeschirren, insbesondere Bett-Pflegegeschirren,

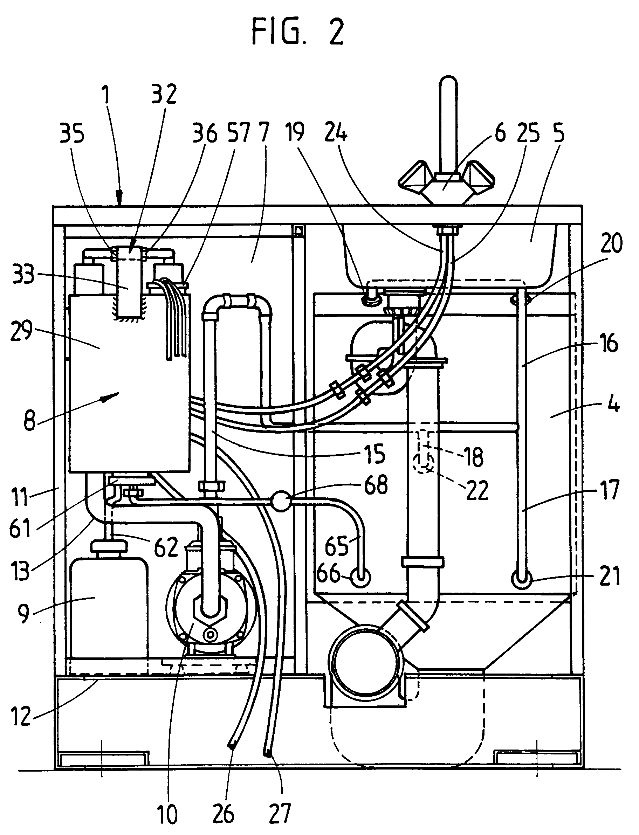

- Figur 2: eine Rückansicht der Vorrichtung, welche einen Wasservorratsbehälter enthält,

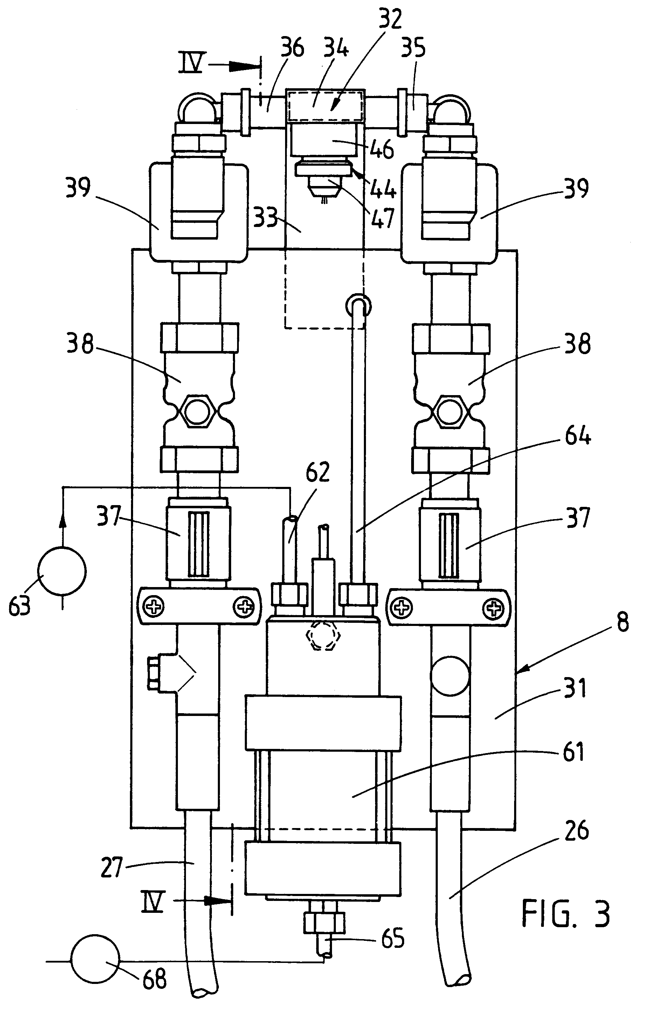

- Figur 3: in Einzeldarstellung eine Ansicht des Wasservorratsbehälters mit Blick auf die Wasseranschlußseite,
- Figur 4: den Schnitt nach der Linie IV-IV in Figur 3,

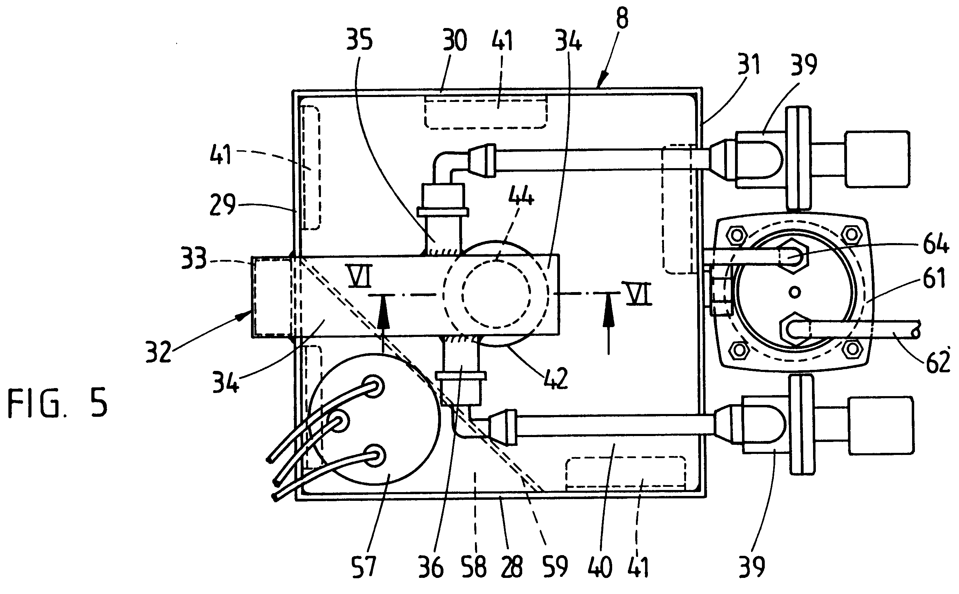

- Figur 5: eine Draufsicht auf den Wasservorratsbehälter,

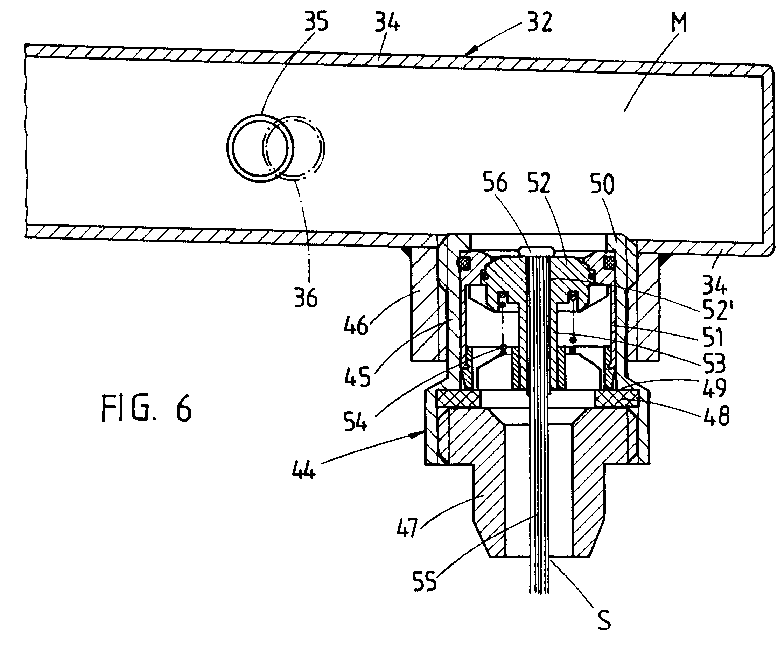

- Figur 6: in vergrößerter Darstellung den Schnitt nach der Linie VI-VI in Figur 5, und zwar bei geschlossenen Absperrventilen,
- Figr 7: eine der Figur 6 entsprechende Darstellung, jedoch bei geöffneten Absperrventilen und durch den Wasserdruck verlagertem, das Borstenbüschel tragendem Ventilteller,

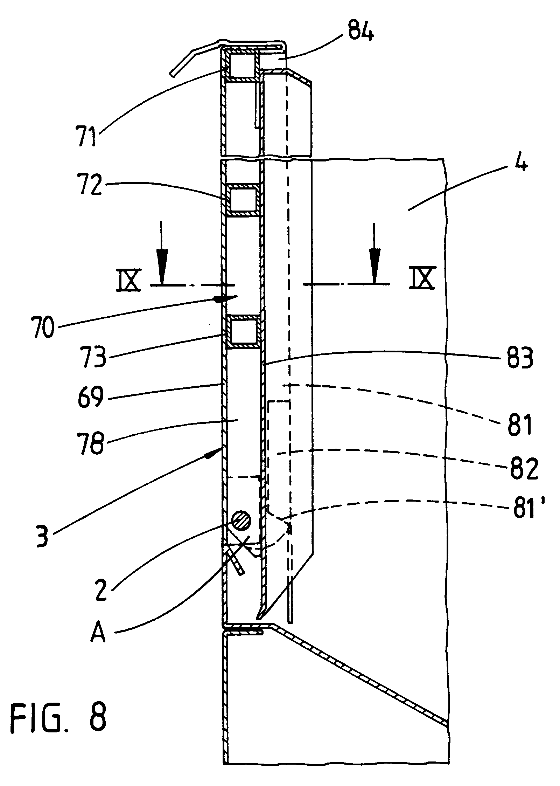

- Figur 8: einen Vertikalschnitt durch die die Kammer verschließende Klappe gemäß ihrer Schließstellung,
- Figur 9: den Schnitt nach der Linie IX-IX in Figur 8,
- Figur 10: eine der Figur 8 entsprechende Darstellung, jedoch bei in die Öffnungsstellung geschwenkter Klappe,
- Figur 11: einen Horizontalschnitt durch die Öffnungsseite der Kammer mit Blick auf die Klappe, wobei das Innenblech der Klappe teilweise weggebrochen dargestellt ist, und
- Figur 12: eine schematische Darstellung der Vorrichtung.

Die dargestellte Vorrichtung besitzt ein spülenartiges Gehäuse 1. Von vorne betrachtet, befindet sich auf der linken Seite des Gehäuses 1 eine um eine untere horizontale Achse 2 schwenkbar angeordnete Klappe 3, welche eine Kammer 4 verschließt. Letztere dient zur Aufnahme von zu reinigenden und desinfizierenden Pflegegeschirren, insbesondere Bett-Pflegegeschirren. Oberhalb der Kammer 4 ist ein Spülbecken 5 vorhanden mit diesem zugeordneter Armatur 6 für Kalt- und Warmwasser.

Die rechte Seite des Gehäuses 1 ist verschließbar durch eine Tür 7. Nach Öffnen derselben sind die verschiedenen Teile der Vorrichtung zugänglich. Bezüglich dieser Teile handelt es sich im wesentlichen um einen Wasservorratsbehälter 8, einen Behälter 9 für Desinfektionsmittel und um eine elektromotorisch betriebene Pumpe 10 zum Spülen.

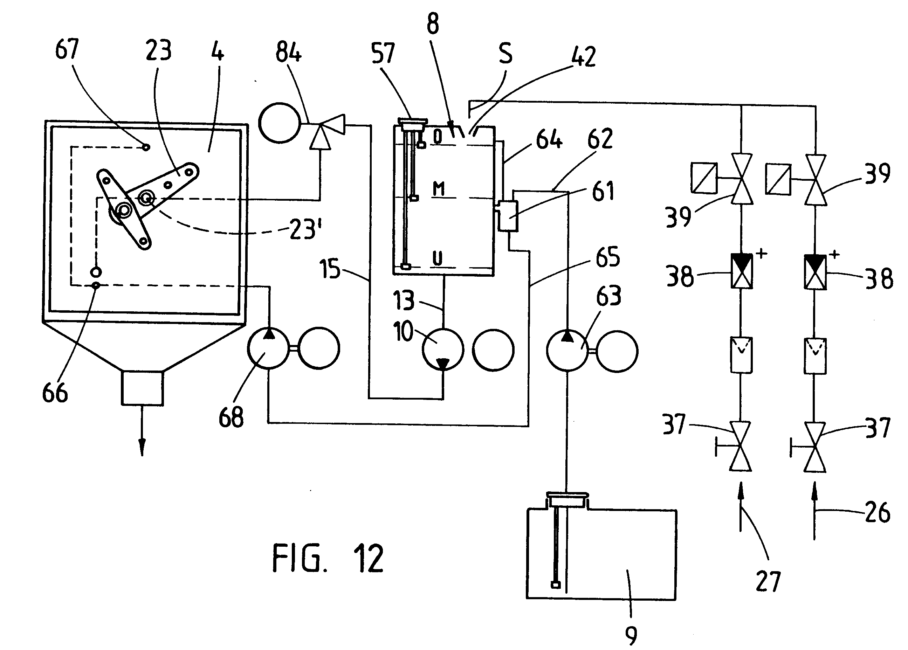

Der Wasservorratsbehälter 8 ist an der einen Gehäuseseitenwand 11 in nicht näher veranschaulichter Weise festgelegt. Beispielsweise kann dort der Wasservorratsbehälter 8 eingehängt sein. Unterhalb des Wasservorratsbehälters 8 befindet sich der das Desinfektionsmittel aufnehmende Behälter 9. Dieser sitzt auf einer Bodenwand 12 des Gehäuses 1 auf. Benachbart zum Behälter 9 ist die Pumpe 10 angeordnet. Diese steht über eine Abflußleitung 13 mit dem Wasservorratsbehälter 8 in Leitungsverbindung. Die Abflußleitung 13 geht aus von dem Boden 14 des Wasservorratsbehälters 8. Mittels der Pumpe 10 wird das dem Wasservorratsbehälter 8 entnommene Spülwasser über eine Förderleitung 15 und ein Umschaltventil 84 Zweigleitungen 16, 17 und 18 zugeführt, welche in der Kammer 4 zugeordneten Einspüldüsen 19, 20, 21 und 22 enden. Der Einspüldüse 22 ist ein in Figur 12 schematisch veranschaulichter Verteiler 23 zugeordnet, welcher in bekannter Weise Ausströmöffnungen besitzt, so daß aufgrund drehbarer Anordnung des Verteilers 23 die Ausspritzdüsen zu einem Drehen des Verteilers 23 um seine Achse 23' führen.

Die Armatur 6 ist an eine Kalt- und Warmwasserleitung 24, 25 angeschlossen. Ebenfalls kann der Wasservorratsbehälter 8 über einen Kaltwasserschlauch 26 und Warmwasserschlauch 27 befüllt werden.

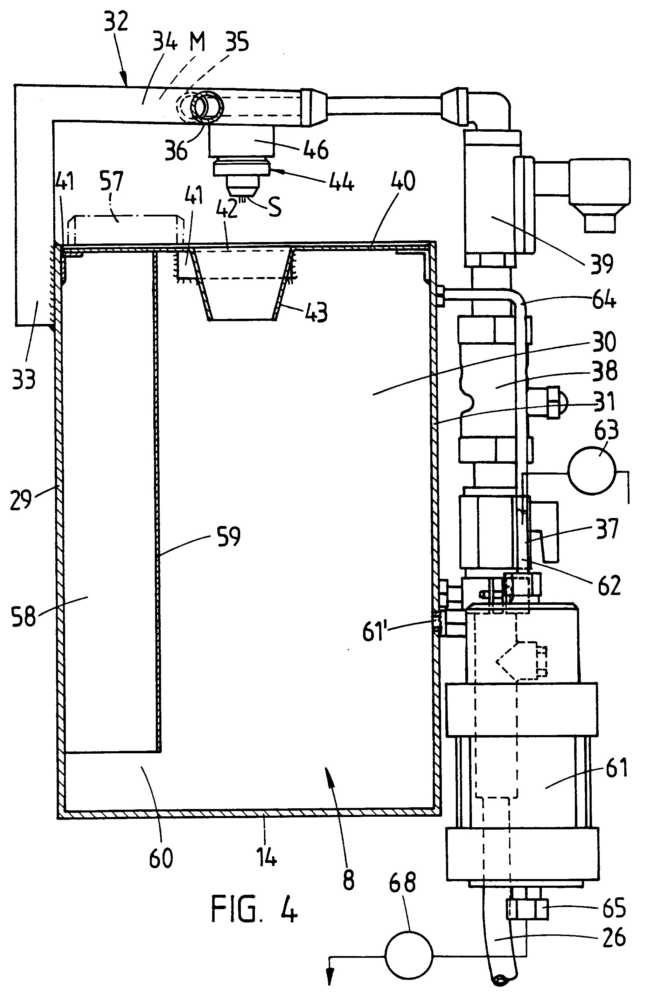

Der Wasservorratsbehälter 8 besitzt einen rechteckförmigen Grundriß. Von dem Boden 14 gehen aufwärtsgerichtet die Behälterwände 28, 29, 30 und 31 aus. Die Behälterwand 29 ist Träger eines sie überfangenden Galgens 32. Letzterer besitzt Winkelform, derart, daß der eine Winkelschenkel 33 am oberen Ende der Behälterwand 29 durch Schweißen festgelegt ist, die Behälterwandung 29 überragt und sich in den anderen Winkelschenkel 34 fortsetzt. Beide Winkelschenkel 33, 34 bestehen aus Vierkanthohlprofil. Das vordere Ende des Winkelschenkels 34 bildet unterseitig eine Strahlaustrittsmündung S aus. Der Innenraum des Winkelschenkels 34 dient als Mischkammer des getrennt zugeführten warmen und kalten Wassers. Deren Zulaufstutzen 35, 36 sind an den Schmalseiten des Vierkanthohlprofiles des Winkelschenkels in Gegenüberlage versetzt zueinander angeordnet, so daß die in den Innenraum eintretende Wasserstrahlen beider Zulaufstutzen 35, 36 sich nicht nachteilig beeinflussen. Wie insbesondere aus Figur 4 ersichtlich ist, verläuft der Winkelschenkel 34 des Galgens in Richtung der Strahlaustrittsmündung S schwach geneigt, was ein restliches Entleeren des Innenraumes begünstigt.

Dem Kalt- und Warmwasserschlauch 26, 27 ist je ein Absperrventil 37, ein nicht veranschauliches Sieb, ein Rückflußverhinderer 38 sowie ein Magnetventil 39 zugeordnet. Von jedem Magnetventil 39 führt eine Leitung zu dem betreffenden Zulaufstutzen 35, 36.

Der Galgen 32 bzw. dessen Winkelschenkel 34 ist so positioniert, daß dessen Strahlaustrittsmündung S über der Decke 40 des Wasservorratsbehälters 8 angeordnet ist. Die Decke 40 ist lose aufgelegt und ruht auf Winkelstücken 41 der Behälterwände. Die unterhalb der Strahlaustrittsmündung S vorgesehene Einflußöffnung 42 in der Decke 40 setzt sich in einen nach innen gerichteten Trichter 43 fort. Dieser wirkt einem Verspritzen des einfließenden Wassers entgegen.

Die Strahlaustrittsöffnung S wird gebildet von einem Ausströmventil 44. Letzteres besitzt ein rohrförmiges Außengehäuse 45, welches in einen Anschlußstutzen 46 an der Unterseite des Winkelschenkels 34 eingeschraubt ist, derart, daß das zugekehrte Stirnende des Außengehäuses 45 bündig mit der Innenwandung der Mischkammer M abschließt.

In das untere Ende des Außengehäuses 45 ist eine aus Teflon bestehende Innenbüchse 47 eingeschraubt. Ihre Materialbeschaffenheit verhindert das Ansetzen von Kalk unter Berücksichtigung unterschiedlicher Wassergualitäten. Unter Zwischenlage eines Dichtungsringes 48 stützt sich die Innenbüchse 47 an einer Innenschulter 49 des Außengehäuses 45 ab. Zwischen der Innenschulter 49 und einem radial einwärts gerichteten Kragen 50 am gegenüberliegenden Ende des Außengehäuses 45 ist eine Büchse 51 eingesetzt, die ihrerseits zur Führung eines Ventiltellers 52 dient. Letzterer setzt sich in einen abwärts gerichteten Ventilschaft 53 fort. Eine Druckfeder 54 umfaßt den Ventilschaft 53 und beaufschlagt den Ventilteller 52 in Verschlußrichtung. Eine zentrale Bohrung 52' durchsetzt den Ventilteller 52 sowie den Ventilschaft 53 und dient zur Aufnahme eines Strahlleitkörpers 55, welcher mit seinem unteren Ende die Innenbüchse 47 und damit die Strahlaustrittsmündung S überragt. Gebildet ist der Strahlleitkörper 55 von einem aus Nylonfäden bestehenden Borstenbüschel, welches an seiner Wurzel 56 zusammengefaßt ist.

Dem Wasservorratsbehälter 8 ist ein Niveauhöhenregler 57 zugeordnet. Zur Unterbringung desselben dient ein abgeteilter Eckbereich 58 des Wasservorratsbehälters 8. Gebildet ist der abgeteilte Eckbereich von einer die Behälterwände 28 und 29 verbindenden Schrägwand 59, die ihrerseits bis zur Decke 40 reicht, jedoch in Bodennähe einen Durchlaßquerschnitt 60 zum abgeteilten Eckbereich 58 beläßt, vgl. insbesondere Figur 4. Mittels des Niveauhöhenreglers 57 können drei Füllstände O, M und U überwacht werden, wobei es sich bezüglich eines oberen, mittleren und unteren Wasserstandes bzw. Niveauspiegels der Flüssigkeit im Wasservorratsbehälter handelt. Dicht unterhalb des mittleren Niveaus ist eine Zumischkammer 61 an der Behälterwand 31 angeflanscht. Oberseitig besitzt diese einen Zufluß 62 für das im Behälter 9 befindliche Desinfektionsmittel. Befördert wird das Desinfektionsmittel aus dem Behälter 9 über eine elektromotorisch betriebene Dosierpumpe 63. In Gegenüberlage zum Zufluß ist ein Überströmanschluß 64 vorgesehen. Durch diesen kann beim Befüllen der Zumischkammer 61 Luft sowie Flüssigkeit in den Wasservorratsbehälter gelangen. Der entsprechende Überströmanschluß mündet hierzu oberhalb des oberen Niveaus O, vgl. insbesondere Figur 12.

Das untere Ende der Zumischkammer 61 steht über einen Abfluß 65 in Leitungsverbindung mit einer Desinfektions-Sprüheinrichtung, welche Einsprühdüsen 66, 67 umfaßt. In den Abfluß 65 eingeschaltet ist eine elektromotorisch angetriebene Einsprühpumpe 68.

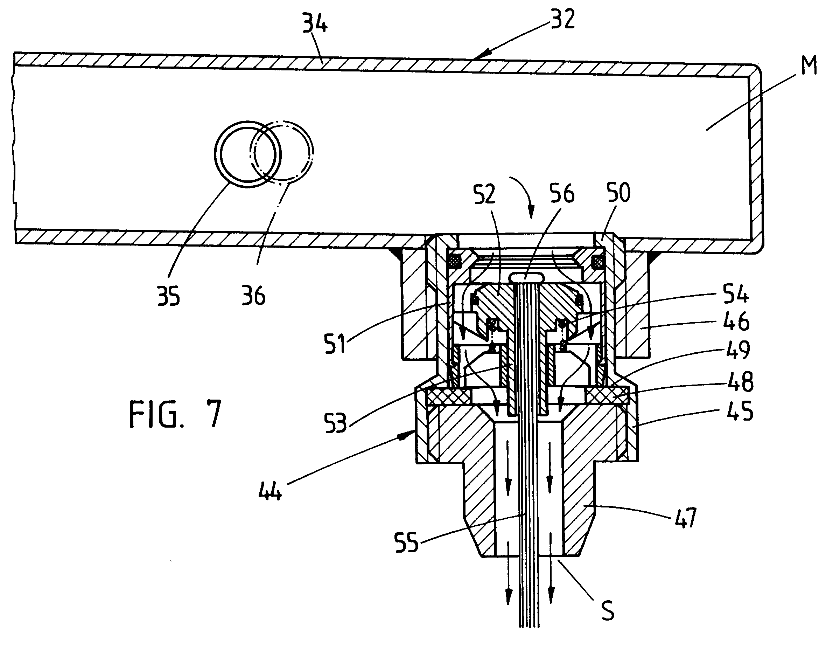

Es stellt sich folgende Wirkungsweise ein: Nach Einbringen des Pflegegeschirrs in die Spülkammer 4 wird der Spülvorgang eingesetzt. Das benötigte Spülwasser wird aus dem Vorratsbehälter 8 über die Pumpe 10 der Spülkammer 4 zugeleitet. Gespeist wird der Vorratsbehälter 8 zunächst mit Kaltwasser, dann mit Kalt/Warmwasser und zuletzt mit Warmwasser, wobei die entsprechenden Mischvorgänge über eine Steuerung geregelt werden können. Dabei steht die Strahlaustrittsmündung S in keiner Flächenverbindung zur wasserbenetzten Innenwand des Wasservorratsbehälters 8, so daß eventuell in diesem befindliche Bakterien nicht in das Wassernetz gelangen können. Während des Wasserzulaufs wird durch den in der Mischkammer M des Galgens 32 herrschenden Wasserdruck der Ventilteller 52 in Abwärtsrichtung gemäß Figur 7 gedrückt. Das durchmischte Wasser umspült den Ventilkörper und wird durch den Strahlleitkörper 55 gebündelt unter Erzielung eines glatten, ein Verspritzen verhindernden Wasserstrahls, welcher gemäß Pfeilrichtung in die Einflußöffnung 42 des Wasservorratsbehälters 8 eintritt. Bei Erreichen eines vorgegebenen Niveaus schließen die betreffenden Absperrventile 37.

Einhergehend mit dem Befüllen des Wasservorratsbehälters 8 wird auch die Zumischkammer 61 gespeist. Liegt der erforderliche Füllstand der Zumischkammer 61 vor, fördert die Dosierpumpe 63 aus dem Behälter 9 die vorgegebene Menge von Desinfektionsmittel in die Zumischkammer 61 hinein. Nach beendetem Spülen des Bett-Pflegegeschirres erfolgt die Desinfektion desselben. Hierzu wird über die Einsprühpumpe 68 die Zumischkammer 61 entleert, wobei die aus der Zumischkammer 61 kommende Desinfektionslösung über die Einsprühdüsen 66, 67 in die Spülkammer 4 geleitet wird.

Anstelle einer dezentralen chemischen Desinfektion, wie vorstehend beschrieben, kann auch eine dezentrale thermische Desinfektion erfolgen. In diesem Falle wäre der Behälter 9 durch einen Dampferzeuger zu ersetzen. Eine Zumischkammer kann in diesem Falle entfallen. Gespeist wird der Dampferzeuger ebenfalls aus dem Vorrat des Wasservorratsbehälters. Nach beendigtem Spülvorgang wird dann der Dampf in die Spülkammer zur Desinfektion geleitet.

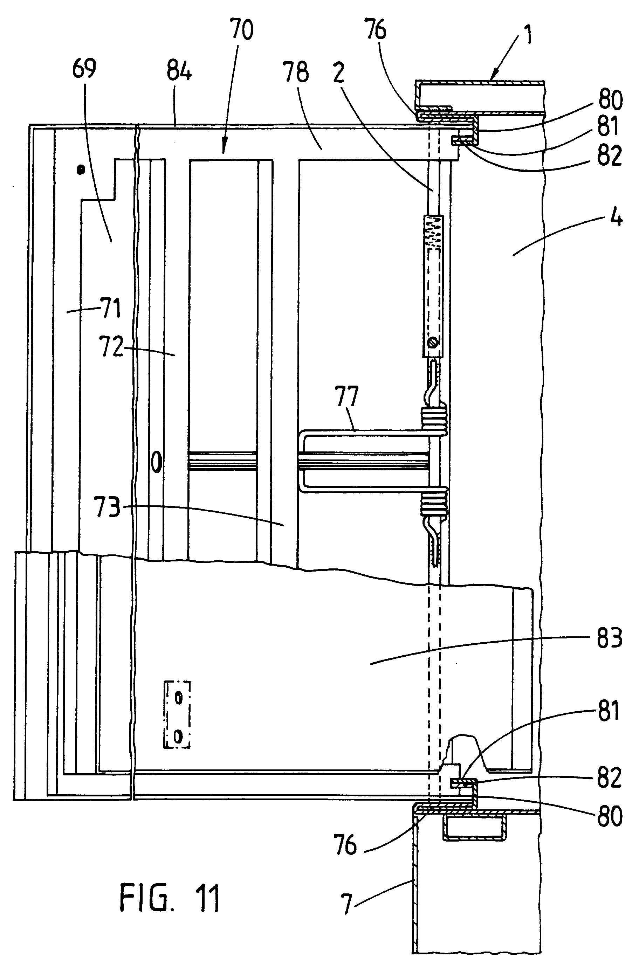

Die die Spülkammer 4 verschließende Klappe 3 besitzt eine Frontblechplatte 69. Diese nimmt einen insbesondere aus Figur 11 hervorgehenden Klappenrahmen 70 auf. Derselbe besteht aus drei parallelen, in der Horizontalen angeordneten Rahmenschenkeln 71, 72 und 73, welche Seitenholme 78, 79 miteinander verbinden. Die Seitenholme 70, 71 stehen dabei über den unteren Rahmenschenkel 73 vor und nehmen am freien Ende die teleskopartig ausgebildete Achse 2 auf, welche drehfest in eine Rand 76 der Spülkammer 4 angeordnet ist. Eine Drehfeder 77 sitzt auf der Achse 2. Durch sie wird die Klappe 3 in Schließrichtung belastet. Die Federkraft ist jedoch so bemessen, daß in der Offenstellung der Klappe 3 kein selbständiges Schließen der Klappe auftritt.

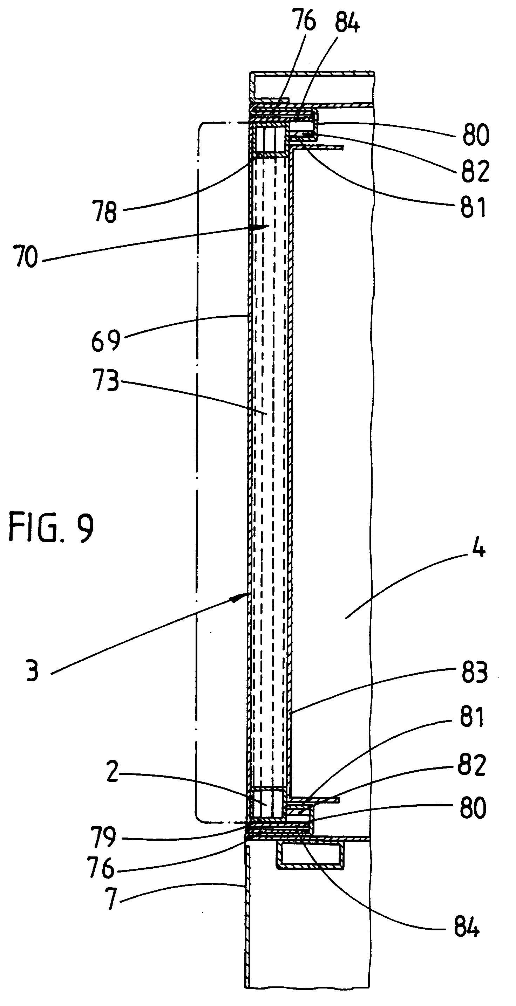

Der Rand 76 setzt sich über einen abgewinkelten Quersteg 80 in eine parallel zu ihm liegende Randeinfassung 81 fort. Zwischen diese und den Rand 76 greift eine abgewinkelte Randleiste 82 der Frontblechplatte 69 in der Verschlußstellung der Klappe ein, vgl. Figur 9.

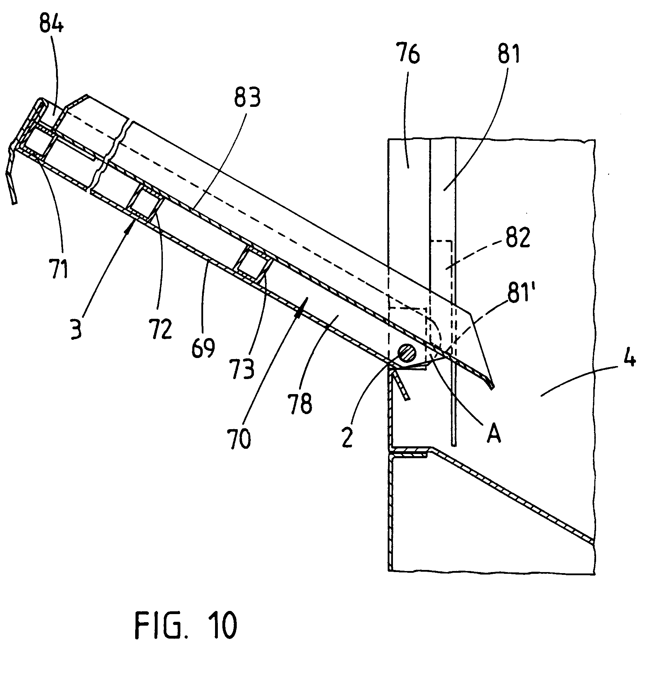

In der Offenstellung der Klappe, vgl. Figur 10, beaufschlagen die über die Achslagerstelle ragenden Anschlagabschnitte A der Seitenholme 78, 79 die untere Schmalkante 81' der Randeinfassung. Im dortigen Bereich sind die Enden der Randeinfassung durch einen aufgesetzten Streifen 82 verstärkt, welcher bis zu den unteren Schmalkanten hin reicht und eine Vergrößerung der Anschlagfläche für die Anschlagabschnitte bildet, so daß auch größere Belastungen schadfrei aufgefangen werden.

Innenseitig der Klappe ist der Klappenrahmen 70 von einer ihn überfangenden Blechplatte 83 abgedeckt, welche parallel verläuft zur Frontblechplatte 69. An der Blechplatte 83 können nicht veranschaulichte Halterungen für Pflegegeschirr etc. vorgesehen sein.

Die in der vorstehenden Beschreibung, der Zeichnung und den Ansprüchen offenbarten Merkmale der Erfindung können sowohl einzeln als auch in beliebiger Kombination für die Verwirklichung der Erfindung von Bedeutung sein. Alle offenbarten Merkmale sind erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen.

## Patentansprüche

1. Vorrichtung zum Spülen und/oder Desinfizieren von Pflegegeschirren, insbesondere Bett-Pflegegeschirren, mit einer das Pflegegeschirr aufnehmenden Kammer und einem dieser vorgeordneten Wasservorratsbehälter mit einer Decke (40), wobei dem Wasservorratsbehälter ein mit dem Wasserleitungsnetz verbindbarer Zulauf zugeordnet ist, und wobei die Strahlaustrittsmündung in keiner Flächenverbindung steht zur wasserbenetzten Innenwand des Wasservorratsbehälters, gekennzeichnet durch einen freien Strahl als Zufluß zum Wasservorratsbehälter (8), derart, daß die Strahlaustrittsmündung (S) über der Decke (40) des Wasservorratsbehälters (8) angeordnet ist und daß die Decke (40) eine Einflußöffnung aufweist.

2. Vorrichtung nach Anspruch 1, dadurch gekennzeichnet, daß die Einflußöffnung (42) der Decke (40) sich als nach innen gerichteter Trichter (43) fortsetzt.

3. Vorrichtung nach einem oder mehreren der vorhergehenden Ansprüche, dadurch gekennzeichnet, daß im Zentrum des Strahles ein Strahlleitkörper (55), vorzugsweise in Form eines Borstenbüschels, angeordnet ist.

4. Vorrichtung nach einem oder mehreren der vorhergehenden Ansprüche, dadurch gekennzeichnet, daß der Strahlleitkörper (55) an einem verschieblichen, vom einfließenden Wasser umspülten Ventilteller (52) sitzt.

5. Vorrichtung nach einem oder mehreren der vorhergehenden Ansprüche, dadurch gekennzeichnet, daß die Strahlaustrittsmündung (S) an der Unterseite eines Galgens (32) angeordnet ist, dessen Innenraum als Mischkammer (M) des getrennt zugeführten warmen und kalten Wassers dient.

6. Vorrichtung nach einem oder mehreren der vorhergehenden Ansprüche, dadurch gekennzeichnet, daß die Zulaufstutzen (35, 36) am Galgen einander entgegengerichtet und aus einer Gegenüberlage versetzt angeordnet sind.

7. Vorrichtung nach einem oder mehreren der vorhergehenden Ansprüche, dadurch gekennzeichnet, daß der Galgen (32) in Richtung der Strahlaustrittsmündung (S) schwach geneigt verläuft.

8. Vorrichtung nach einem oder mehreren der vorhergehenden Ansprüche, gekennzeichnet durch einen Niveauhöhenregler (57) im Wasservorratsbehälter.

9. Vorrichtung nach einem oder mehreren der vorhergehenden Ansprüche, dadurch gekennzeichnet, daß der Niveauhöhenregler (57) in einem abgeteilten Eckbereich (58) des Wasservorratsbehälters (8) angeordnet ist, der in Bodennähe einen Durchlaßquerschnitt (60) zum abgeteilten Eckbereich (58) beläßt.

10. Vorrichtung nach einem oder mehreren der vorhergehenden Ansprüche, dadurch gekennzeichnet, daß am Wasservorratsbehälter (8) vorzugsweise auf Höhe eines mittleren gesteuerten Niveaus eine Zumischkammer (61) angeflanscht ist, welche einen Zufluß (62) für ein Desinfektionsmittel besitzt und einen Abfluß (65), der zu einer in der Spülkammer (4) vorgesehenen Desinfektions-Sprüheinrichtung (Einsprühdüsen 66, 67) führt.

11. Vorrichtung nach einem oder mehreren der vorhergehenden Ansprüche, dadurch gekennzeichnet, daß die in waagerechte Richtung öffenbare Klappe (3) der Kammer (4) achsgelagerte Seitenholme (78, 79) besitzt, die über die Achslagerstelle ragende Anschlagabschnitte (A) ausbilden, welche gegen die untere Schmalkante (81') einer Randeinfassung (81) der frontseitigen Öffnung der Kammer (4) schwenken.

12. Vorrichtung nach einem oder mehreren der vorhergehenden Ansprüche, dadurch gekennzeichnet, daß die Randeinfassung (81) einen Einschwenkquerschnitt für eine Randleiste (82) der Klappe (3) ausbildet und, bis zu den Schmalkanten (81') reichend, an den freien unteren Enden verstärkt ist.

13. Vorrichtung nach einem oder mehreren der vorhergehenden Ansprüche oder insbesondere danach, dadurch gekennzeichnet, daß die Seitenholme (78, 79) Bestandteil eines Klappenrahmens (70) sind.

## Claims

1. Apparatus for washing and/or disinfecting care utensils, in particular bed care utensils, with a chamber receiving the care utensils and in front of it a water storage tank with a cover (40), wherein associated with the water storage tank is a supply which can be connected to the water main, and wherein the jet outlet opening has no surface connection with the water-wetted inner wall of the water storage tank, characterised by a free jet as the inflow to the water storage tank (8), such that the jet outlet opening (S) is arranged above the cover (40) of the water storage tank (8), and in that the cover (40) comprises an inflow opening.

2. Apparatus according to claim 1, characterised in that the inflow opening (42) of the cover (40) continues as an inwardly directed funnel (43).

3. Apparatus according to one or more of the preceding claims, characterised in that at the centre of the jet is arranged a jet conducting body (55), preferably in the form of a bundle of bristles.

4. Apparatus according to one or more of the preceding claims, characterised in that the jet conducting body (55) is mounted on a slidable valve plate (52) around which the inflowing water circulates.

5. Apparatus according to one or more of the preceding claims, characterised in that the jet outlet opening (S) is arranged on the lower side of a cross-beam (32) of which the interior serves as a mixing chamber (M) for the separately supplied hot and cold water.

6. Apparatus according to one or more of the preceding claims, characterised in that the supply connection pieces (35, 36) on the cross-beam point in opposite directions to each other and are offset from opposition.

7. Apparatus according to one or more of the preceding claims, characterised in that the cross-beam (32) is slightly inclined in the direction of the jet outlet opening (S).

8. Apparatus according to one or more of the preceding claims, characterised by a level regulator (57) in the water storage tank.

9. Apparatus according to one or more of the preceding claims, characterised in that the level regulator (57) is arranged in a partitioned corner region (58) of the water storage tank (8) which in the vicinity of the bottom leaves a passage cross-section (60) from the partitioned corner region (58).

10. Apparatus according to one or more of the preceding claims, characterised in that onto the water storage tank (8) is preferably flanged, at the height of a medium controlled level, an adding chamber (61) which has an inflow (62) for a disinfectant and an outflow (65) which leads to a disinfecting sprayer (spray nozzles 66, 67) provided in the wash chamber (4).

11. Apparatus according to one or more of the preceding claims, characterised in that the door (3) of the chamber (4), which can be opened in a horizontal direction, has axle-mounted side members (78, 79) forming stop sections (A) which extend beyond the axle mounting point and which pivot towards the lower narrow edge (81') of an edge surround (81) of the front opening of the chamber (4).

12. Apparatus according to one or more of the preceding claims, characterised in that the edge surround (81) forms a cross-section for inward pivoting of an edge strip (82) of the door (3) and, extending up to the narrow edges (81'), is reinforced at the free lower ends.

13. Apparatus according to one or more of the preceding claims or in particular according thereto, characterised in that the side members (78, 79) form part of a door frame (70).

## Revendications

1. Dispositif pour laver et/ou désinfecter des utensiles de soins, en particulier des bassins hygiéniques pour personnes alitées, avec une chambre recevant l'utensile de soins et un réservoir de stockage d'eau disposé en amont de celle-ci, avec un plafond (40), une alimentation susceptible d'être reliée au réseau d'eau étant associée au réservoir de stockage d'eau, et l'embouchure de sortie de jet n'étant pas en liaison superficielle avec la paroi intérieure mouillée par l'eau du récipient de stockage d'eau, caractérisé par un jet libre faisant office de source d'alimentation pour le réservoir de stockage d'eau (8), de telle manière que l'embouchure de sortie de jet (S) soit disposée au-dessus du plafond (40) du réservoir de stockage d'eau (8) et en ce que le plafond (40) comporte une ouverture d'admission.

2. Dispositif selon la revendication 1, caractérisé en ce que l'ouverture d'admission (42) du plafond (40) se prolonge sous la forme d'un entonnoir (43) dirigé vers l'intérieur.

3. Dispositif selon une ou plusieurs des revendications précédentes, caractérisé en ce qu'au centre du jet est disposé a corps de guidage de jet (55), se présentant de préférence sous la forme d'un faisceau de fils raides (de brosse).

4. Dispositif selon une ou plusieurs des revendications précédentes, caractérisé en ce que le corps de guidage de jet (55) est placé sur un clapet de valve coulissant (52), balayé par l'écoulement de l'eau entrante.

5. Dispositif selon une ou plusieurs des revendications précédentes, caractérisé en ce que l'embouchure de sortie de jet (S) est disposée sur la face inférieure d'une potence (32), dont l'espace intérieur sert de chambre de mélange (M) pour l'eau chaude et l'eau froide amenées séparément.

6. Dispositif selon une ou plusieurs des revendications précédentes, caractérisé en ce que les raccords ou tubulures d'amenée (35, 36) sont disposés sur la potence orientés en sens inverse l'un par rapport à l'autre, et décalés de façon à ne pas se trouver exactement l'un en face de l'autre.

7. Dispositif selon une ou plusieurs des revendications précédentes, caractérisé en ce que la potence (32) est disposée légèrement inclinée dans la direction de l'embouchure de sortie de jet (S).

8. Dispositif selon une ou plusieurs des revendications précédentes, caractérisé par a régulateur de hauteur de niveau (57) dans le réservoir de stockage d'eau.

9. Dispositif selon une ou plusieurs des revendications précédentes, caractérisé en ce que le régulateur de hauteur de niveau (57) est disposé dans une zone d'angle compartimentée (58) du réservoir de stockage d'eau (8), en laissant subsister à proximité du fond une section transversale de passage (60), vis-à-vis de la zone d'angle compartimentée (58).

10. Dispositif selon une ou plusieurs des revendications précédentes, caractérisé en ce que sur le récipient de stockage d'eau (8) est flasquée, de préférence à la hauteur d'un niveau contrôlé moyen, une chambre de mélange (61) qui présente une amenée (62) pour un produit de désinfection et une évacuation (65) conduisant à un organe de pulvérisation de désinfection (buses d'introduction par pulvérisation 66, 67) prévu dans la chambre de lavage (4).

11. Dispositif selon une ou plusieurs des revendications précédentes, caractérisé en ce que le volet (3) de la chambre (4), susceptible d'être ouvert dans la direction horizontale, comporte des montants latéraux (78, 79) tourillonnant sur un axe et qui constituent des tronçons de butée (A) faisant saillie au-delà du point de tourillonnement d'axe et pivotent contre l'arête étroite inférieure (81') d'une monture de bordure (81) de l'ouverture frontale de la chambre (4).

12. Dispositif selon me ou plusieurs des revendications précédentes, caractérisé en ce que la monture de bordure (81) constitue une section transversale de pivotement pour une barrette de bordure (82) du volet (3) et qui, en s'étendent jusqu'aux arêtes étroites (81'), est renforcée aux extrémités inférieures libres.

13. Dispositif selon ou en particulier selon une ou plusieurs des revendications précédentes, caractérisé en ce que les montants latéraux (78, 79) font partie d'un cadre de volet (70).
